# EUROPEAN PATENT APPLICATION

(11) **EP 3 821 781 A1**
(43) Date of publication of application: **19.05.2021**
(21) Application number: 19208458.0
(22) Date of filing: 12.11.2019
(51) Int. Cl.: A61B 1/00, A61B 1/005

(54) **ENDOSCOPE WITH INSPECTION ACCESSORY**

(71) Applicant: Tseng, Ching-Shun, Kaohsiung City (TW)
(72) Inventor: Tseng, Ching-Shun, Kaohsiung City (TW)
(74) Representative: Becker & Kurig Partnerschaft Patentanwälte PartmbB

(57) **Abstract**

An endoscope has an inspection accessory (2A) and an operating set (3A). The inspection accessory (2A) has a distal end cap (11), a fastening component (14A) connected to the operating set (3A), a flexible shaft (30A), an objective optical set (10) mounted within the distal end cap (11) and the flexible shaft (30A), a bending mechanism (20) disposed within the flexible shaft (30A) and connected to the distal end cap (11), and a sheath (40) sleeved on the flexible shaft (30A) and having two opposite ends respectively tightly connected to the distal end cap (11) and the fastening component (14A). The flexible shaft (30A) is telescopic, flexible, and hollow and has a first end connected to the fastening component (14A) and a second end opposite to the first end of the flexible shaft (30A) and connected to the distal end cap (11).

## Description

### 1. Field of the Invention

The present invention relates to a medical instrument, and more particularly to an endoscope with an inspection accessory that can be smoothly inserted into a human body.

### 2. Description of Related Art

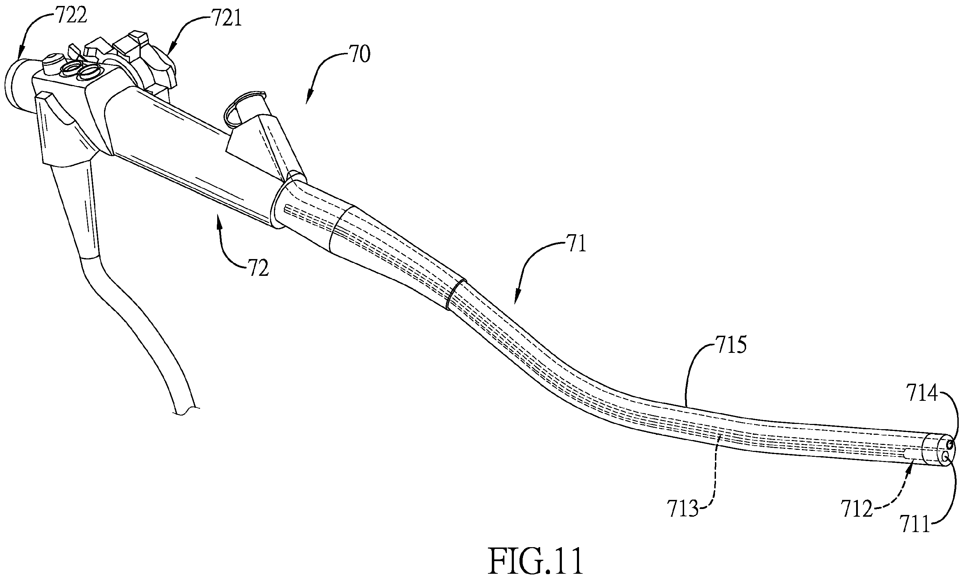

An endoscope is widely applied to gastroscopy, colonoscopy, nephroscopy, and so on. The endoscope is a medical instrument for intruding into the human body for inspection. With reference to Fig. 11, a conventional endoscope 70 applied to colonoscopy has an endoscope accessory 71 and an operating set 72. The endoscope accessory 71 has an objective optical set 711, a bending mechanism 712, multiple angulation wires 713, a conducting tube 714, and a solid shaft 715. The objective optical set 711, the bending mechanism 712, the multiple angulation wires 713, and the conducting tube 714 are enclosed in the solid shaft 715. The solid shaft 715 is difficult to bend. The operating set 72 has a processing unit, a wire operating unit 721, and a viewing device 722.

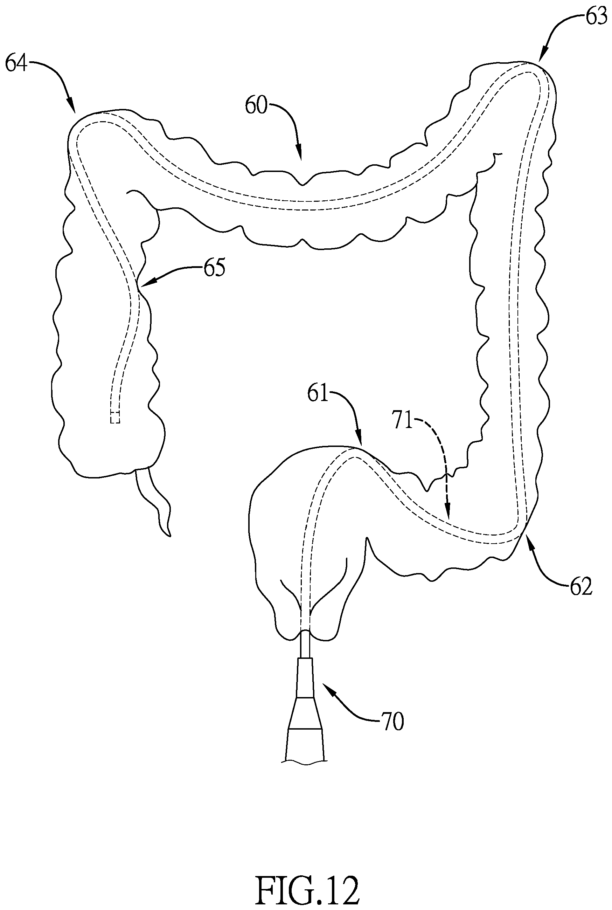

With reference to Fig. 12, when the solid shaft 715 intrudes into a large intestine 60 and bends according to the large intestine 60, a composition force is formed at a bended portion of the solid shaft 715 and acts toward the intestine 60. The composition force formed at the bended portion of the solid shaft 715 drives the bended portion of the solid shaft 715 to abut against the large intestine 60. The bended portion of the solid shaft 715 abutting against the large intestine 60 may cause colonoscopic perforations. With reference to Fig. 12, the large intestine 60 has four curved portions 61, 62, 63, 64. Therefore, the colonoscopic perforations may occur at the four curved portions 61, 62, 63, 64 of the intestine 60 when the solid shaft 715 passes through the four curved portions 61, 62, 63, 64. The incidence of colonoscopic perforations ranges from three thousandths to five thousandths following colonoscopies.

Consequently, the conventional endoscope 70 with the solid shaft 715 is difficult to operate and is likely to cause colonoscopic perforations of the large intestine 60 or perforations of a wall of other organs. How to keep the endoscope smoothly moving inside the human body and bending neatly is a critical issue.

Moreover, the conventional endoscope 70 is too expensive to be disposable. The objective optical set 711, the bending mechanism 712, the multiple angulation wires 713, and the conducting tube 714 enclosed in the solid shaft 715 are difficult to be disinfected by standard disinfection procedure. Even the most rigorous disinfection procedure cannot guarantee the disinfected endoscope is sterile. There is still a risk of complications caused by bacteria or virus contamination. Therefore, how to prevent complications caused by bacteria or virus contamination during endoscopy is another critical object.

To overcome the shortcomings of the conventional endoscope, the present invention provides an endoscope with an inspection accessory to mitigate or obviate the aforementioned problems.

The main objective of the present invention is to provide an endoscope with an inspection accessory that is safer to use and is sterile.

The endoscope comprises an inspection accessory and an operating set. The inspection accessory has a distal end cap, a fastening component, a flexible shaft, an objective optical set, a bending mechanism, and a sheath. The fastening component is connected to the operating set. The flexible shaft is telescopic, flexible, and hollow, and has a first end connected to the fastening component and a second end opposite to the first end of the flexible shaft and connected to the distal end cap. The objective optical set is mounted within the distal end cap and the flexible shaft. The bending mechanism is disposed within the flexible shaft and is connected to the distal end cap. The sheath is sleeved on the flexible shaft and has two opposite ends respectively tightly connected to the distal end cap and the fastening component.

Other objects, advantages, and novel features of the invention will become more apparent from the following detailed description when taken in conjunction with the accompanying drawings.

### In the drawings:

Fig. 1 is a perspective view of a first embodiment of an endoscope in accordance with the present invention, showing a flexible shaft of a first configuration is connected to a bending component of a bending mechanism;
Fig. 2 is a perspective view of a second embodiment of an endoscope in accordance with the present invention, showing the flexible shaft of the first configuration is connected to a distal end cap;
Fig. 3 is a perspective view of a third embodiment of an endoscope in accordance with the present invention, showing the flexible shaft of the first configuration is connected to the bending component of the bending mechanism and a wire operating unit is detachably connected to a main body;
Fig. 4 is an exploded perspective view of the endoscope in Fig. 1, showing the flexible shaft of the first configuration;
Fig. 5 is an exploded perspective view of the endoscope in Fig. 1, showing a flexible shaft of a second configuration;
Fig. 6 is an exploded perspective view of the endoscope in Fig. 1, showing a flexible shaft of a third configuration;
Fig. 7 is an enlarged schematic side view of the flexible shaft in Fig. 6, showing multiple elemental strips of the flexible shaft connected in sequence;
Fig. 8 is a side view in partial section of the endoscope in Fig. 1;
Fig. 9 is a side view in partial section of the endoscope in Fig. 2;
Fig. 10 is an operational side view in partial section of the endoscope in Fig. 2, showing the distal end cap sweeps;
Fig. 11 is a perspective view of an endoscope in accordance with the prior art; and
Fig. 12 is an operational side view of the conventional endoscope in Fig. 11, showing the conventional endoscope is inserted in a large intestine of a human body.

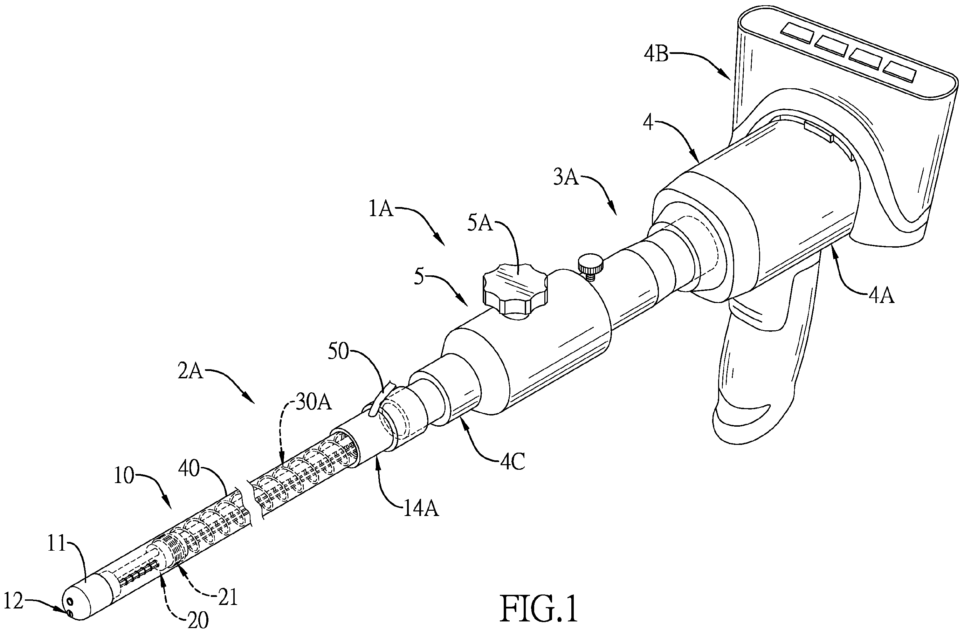

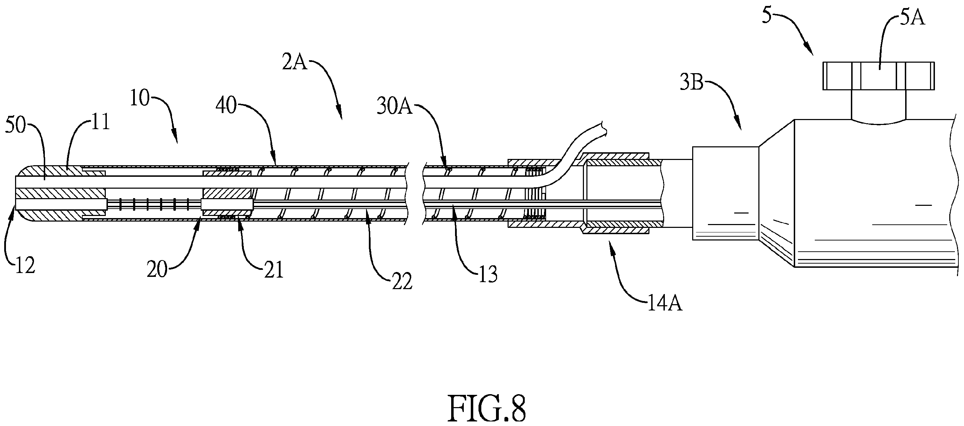

With reference to Figs. 1, 4, and 8, a first embodiment of the endoscope 1A with an inspection accessory 2A in accordance with the present invention has the inspection accessory 2A and an operating set 3A. The inspection accessory 2A is detachably connected to the operating set 3A.

With reference to Figs. 1, 4, and 8, the operating set 3A has a main body 4 and a wire operating unit 5. The main body 4 has a base 4A, a processing unit, a display 4B, a handle, a connecting portion 4C, and a connector. The base 4A has a front end, a rear end, and a lower end. The front end and the rear end of the base 4A are opposite to each other. The processing unit is disposed within the base 4A. The display 4B is disposed at the rear end of the base 4A. The handle is disposed at the lower end of the base 4A for holding. The connecting portion 4C is disposed at the front end of the base 4A. The connector is disposed within the connecting portion 4C. The connector and the display 4B are electrically connected to the processing unit. The wire operating unit 5 has a knob 5A.

With reference to Figs. 1, 4, and 8, the inspection accessory 2A has a distal end cap 11, a fastening component 14A, a flexible shaft 30A, an objective optical set 10, a bending mechanism 20, a sheath 40, and a conducting tube 50. The fastening component 14A is connected to the connecting portion 4C of the main body 4.

With reference to Figs. 1, 4, and 8, showing the flexible shaft 30A of a first configuration. The flexible shaft 30A is telescopic, flexible, and hollow and has a first end and a second end opposite to the first end of the flexible shaft 30A. The flexible shaft 30A is constructed by multiple elemental parts connected in sequence. Each one of the multiple elemental parts is capable of moving relative to each other.

The first end of the flexible shaft 30A is connected to the fastening component 14A. The flexible shaft 30A may be made of steel or plastic. In the first embodiment of the inspection accessory 2A, the flexible shaft 30A of the first configuration is made up of a spiral thread in a configuration of a compression spring and has an outside diameter. The multiple elemental parts are multiple coils separately disposed. Each one of the multiple coils is capable of moving relative to one another. More specifically, the flexible shaft 30A is a compression spring that is easily accessible and low-priced.

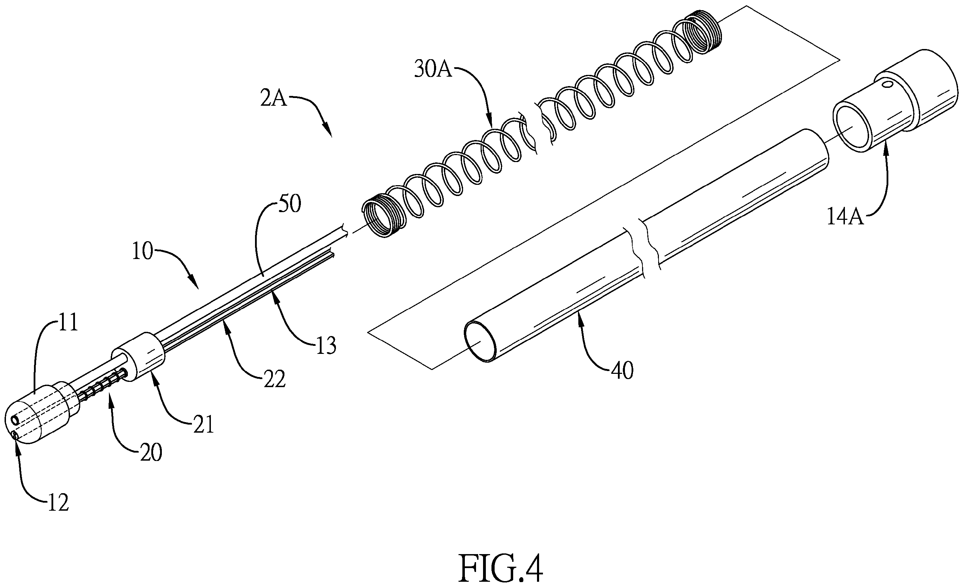

With reference to Fig. 4, the multiple coils are separately disposed and do not contact one another. The size of the flexible shaft 30A is designed according to various endoscopes for various purposes. The spiral thread has a thread diameter. A pitch is defined between each two adjacent coils of the multiple coils of the flexible shaft 30A. For the endoscope applied to colonoscopy, the outside diameter of the flexible shaft 30A is greater than or equal to 8 millimeters and is less than or equal to 14 millimeters, the thread diameter is greater than or equal to 0.5 millimeters and is less than or equal to 1 millimeter, and the pitch is greater than or equal to 1 millimeter and is less than or equal to 10 millimeters. In the first embodiment of the inspection accessory 2A, since the multiple coils are separately disposed, a lateral surface area of the flexible shaft 30A is reduced, and frequency of the multiple coils rubbing inside of the human body is reduced.

With reference to Figs. 1, 4, and 8, the objective optical set 10 has an objective optical unit 12, a connector element, and a cable set 13. The optical unit 12 is mounted within the distal end cap 11. The optical unit 12 has an image sensor for capturing images and an illumination unit for illumination. The image sensor may be a charge-coupled device (CCD) or a complementary metal-oxide semiconductor (CMOS). The illumination unit may be a light-emitting diode (LED). The connector element is disposed within the fastening component 14A and is electrically connected to the connector of the main body 4. The cable set 13 is electrically connected to the image sensor and the illumination unit of the objective optical unit 12, extends within the flexible shaft 30A, and extends into the fastening component 14A to connect the connector element. The cable set 13 has a signal cable and a power cable for transmitting signals and current, respectively. Images captured by the objective optical unit 12 are transmitted via the cable set 13 to the connector element of the objective optical set 10. Then, the images are transmitted to the processing unit of the main body 4 via the connector of the main body 4. Eventually, the images are shown on the display 4B.

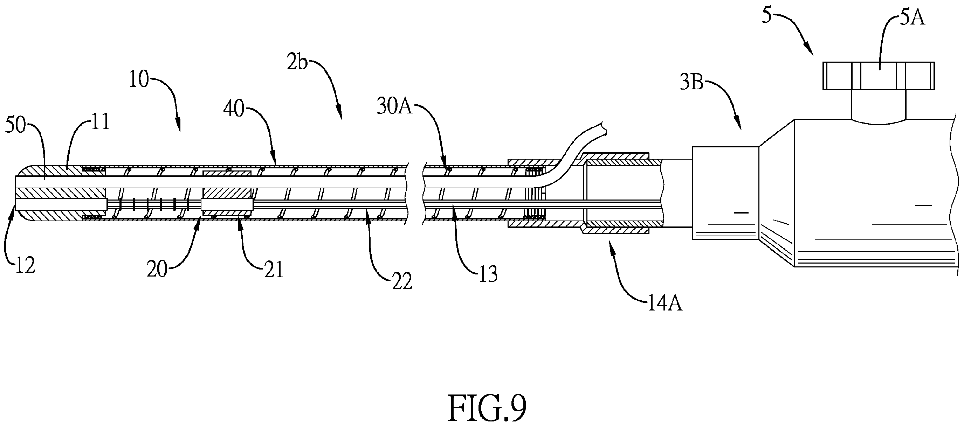

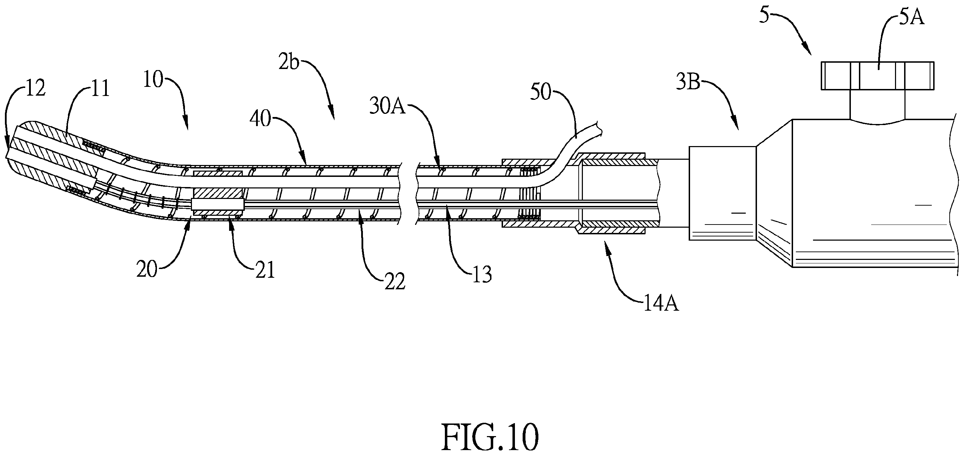

With reference to Figs. 1, 4, and 8, the bending mechanism 20 has a bending component 21 and multiple angulation wires 22. The bending component 21 is disposed within the flexible shaft 30A. Each one of the multiple angulation wires 22 has two opposite ends. One of the two opposite ends of each one of the multiple angulation wires 22 passes through the bending component 21 and is connected to the objective optical unit 12. The other one of the two opposite ends of each one of the multiple angulation wires 22 is connected to the knob 5A. The bending mechanism 20 is connected to the distal end cap 11 via the objective optical unit 12 mounted inside the distal end cap 11. With reference to Figs. 9 and 10, when the knob 5A is rotated, the multiple angulation wires 22 are driven by the knob 5A, and the multiple angulation wires 22 drive the distal end cap 11 to direct the inspection accessory 2A. In the first embodiment of the inspection accessory 2A, the second end of the flexible shaft 30A is connected to the bending component 21.

With reference to Figs. 1, 4, and 8, the sheath 40 is biocompatible and waterproof. The sheath 40 is sleeved on the flexible shaft 30A, fits the flexible shaft 30A, and has two opposite ends, an inner surface, and a thickness. One of the two opposite ends of the sheath 40 is tightly connected to the distal end cap 11. The other one of the two opposite ends of the sheath 40 is tightly connected to the fastening component 14A. The inner surface of the sheath 40 contacts the flexible shaft 30A. The sheath 40 is highly flexible without interfering with movements of the flexible shaft 30A. In the first embodiment of the inspection accessory 2A, the thickness of the sheath 40 is greater than or equal to 0.01 millimeters and is less than or equal to 0.1 millimeters. The sheath 40 is made of easily accessible and low-priced materials, and is made in a sterile environment to avoid contamination.

With reference to Figs. 1, 4, and 8, the conducting tube 50 is disposed within the flexible shaft 30A and has a front end and a rear end. The front end and the rear end of the conducting tube 50 are opposite to each other. The front end of the conducting tube 50 is mounted in the distal end cap 11 and communicates with an exterior of the distal end cap 11. The rear end of the conducting tube 50 extends out of the fastening component 14A and communicates with a fluid supplier for supplying water and air. The conducting tube 50 may feed air for inflating to facilitate insertion of the inspection accessory 2A. The conducting tube 50 also may feed water for cleaning or irrigation to promote quality of images captured by the objective optical set 10. The conducting tube 50 may also be applied for suction and removal of fluid. The conducting tube 50 may allow a biopsy instrument to pass and to extract sample cells or tissues as well. The biopsy instrument may be biopsy forceps, a biopsy needle, or even a flexible metal wire.

Before insertion of the inspection accessory 2A into a large intestine, lubricant shall be applied on the distal end cap 11 and the sheath 40. An endoscopist holds the main body 4 of the operating set 3A with one hand and inserts the inspection accessory 2A into the large intestine with the other hand. The images captured by the objective optical unit 12 are shown on the display 4B.

The conducting tube 50 may inject air to enlarge the large intestine for easily inserting the inspection accessory 2A. The conducting tube 50 may alternatively inject water to wash inside of the large intestine for clearly capturing the images.

The flexible shaft 30A of the first configuration has multiple coils disposed separately. When the flexible shaft 30A bends, the multiple coils are capable of moving separately from one another at bending portions of the flexible shaft 30A without contacting one another and make the flexible shaft 30A capable of bending easily. Therefore, the inspection accessory 2A with the flexible shaft 30A smoothly passes through curved portions of the large intestine without abutting against the large intestine intensely. The flexible shaft 30A not only facilitates the inspection accessory 2A to pass through the curved portions of the large intestine gently, but also provides a buffering function when the inspection accessory 2A contacts the large intestine. Compared to the conventional endoscope 70 with the solid shaft 715, the inspection accessory 2A with the flexible shaft 30A further reduces the incidence of colonoscopic perforations.

The flexible shaft 30A being a compression spring is easily accessible and low-priced and can reduce manufacturing expense of the inspection accessory 2A. Therefore, the inspection accessory 2A with the flexible shaft 30A may be disposed after each colonoscopy without incurring too much cost of disposal. The inspection accessory 2A being disposable does not need to be disinfected before each colonoscopy and can dramatically lower down incidence of infection. The inspection accessory 2A with little cost of disposal makes colonoscopy affordable for people and widely popularized. The inspection accessory 2A is detachably connected to the operating set 3A by the fastening component 14A. When the inspection accessory 2A is detached from the operating set 3A, the objective optical set 10, the bending mechanism 20, the flexible shaft 30A, the sheath 40, and the conducting tube 50 can be disposed of. Since the sheath 40 is made of low-priced materials, cost of the disposal can further be lowered down. Therefore the inspection accessory 2A in accordance with the present invention reduces expense of disinfection, reduces cost of disposal, and prevents contamination caused by bacteria and virus.

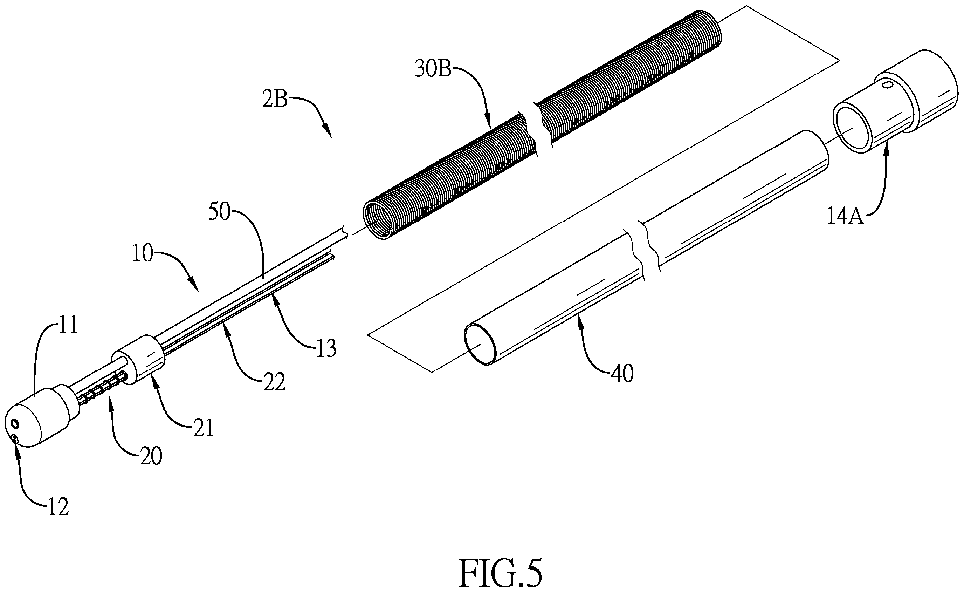

With reference to Fig. 5, the flexible shaft 30B of a second configuration is shown, which is made up of a spiral thread in the configuration of an extension spring.

The multiple elemental parts of the flexible shaft 30B are multiple coils tightly disposed and abutting one another. The outside diameter of the flexible shaft 30B is greater than or equal to 8 millimeters and is less than or equal to 14 millimeters and the thread diameter is greater than or equal to 0.5 millimeters and is less than or equal to 1 millimeter. More specifically, the flexible shaft 30B is an extension spring.

Since the flexible shaft 30B of the second configuration has multiple coils disposed tightly, when the flexible shaft 30B is inserted into the large intestine, the multiple coils abut one another and make the inspection accessory 2B easily controlled and easily inserted into the large intestine. Similarly, when the flexible shaft 30B passes through the curved portions of the large intestine, the flexible shaft 30B bends, the multiple coils are capable of moving separately from one another at bending portions of the flexible shaft 30B without contacting one another and also make the flexible shaft 30B capable of bending easily.

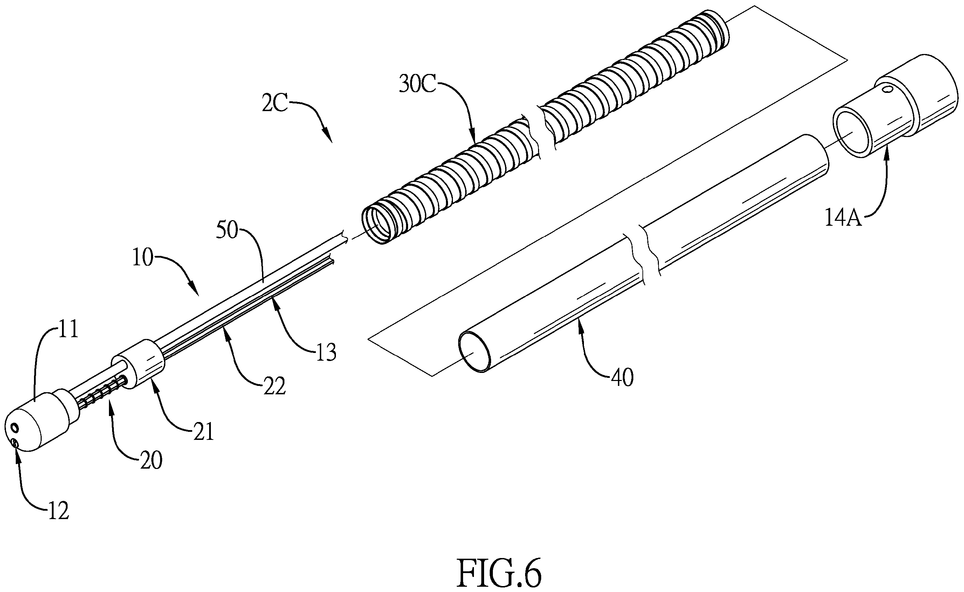

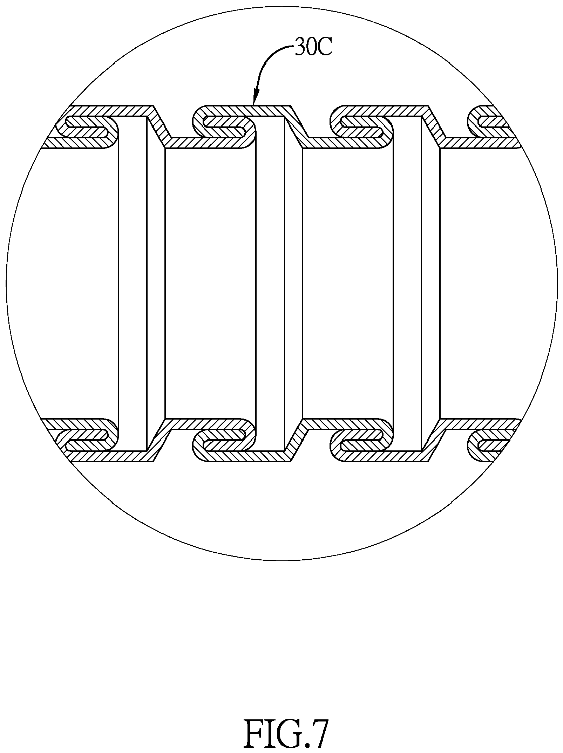

With reference to Figs. 6 and 7, showing the flexible shaft 30C of a third configuration. The flexible shaft 30C is also made up of multiple parts connected in sequence. More specifically, the flexible shaft 30C is a flexible conduit with multiple elemental strips connected continuously. The multiple elemental strips are sequentially connected to one another and capable of moving relative to one another. The flexible shaft 30C may be even made up of a spiral sheet. The outside diameter of the flexible shaft 30C is greater than or equal to 8 millimeters and is less than or equal to 14 millimeters. Each one of the multiple elemental strips or the spiral sheet has a thickness. The thickness of each one of the multiple elemental strips or the spiral sheet is greater than or equal to 0.5 millimeters and less than or equal to 0.8 millimeters.

Since the flexible shaft 30C of the third configuration has multiple elemental strips connected in sequence and capable of moving relative to one another, the inspection accessory 2C can be gently and smoothly inserted into the large intestine as well.

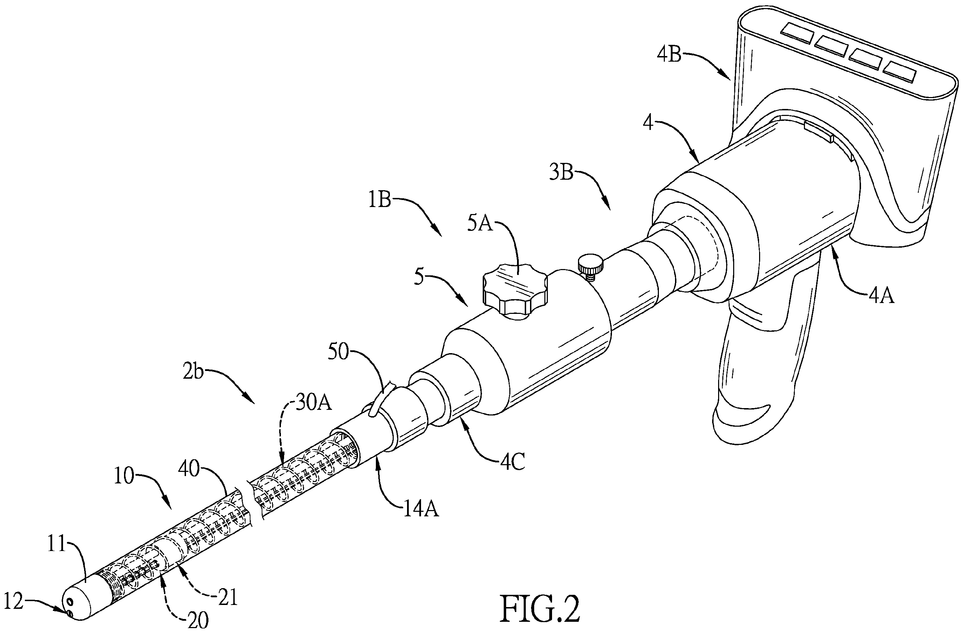

With reference to Fig. 2, a second embodiment of the endoscope 1B has the inspection accessory 2b and the operating set 3B. In the second embodiment, the inspection accessory 2b is substantially same as the inspection accessory 2A of the first embodiment. In the second embodiment, the flexible shaft 30A is connected to the distal end cap 11 rather than the bending component 21 of the bending mechanism 20.

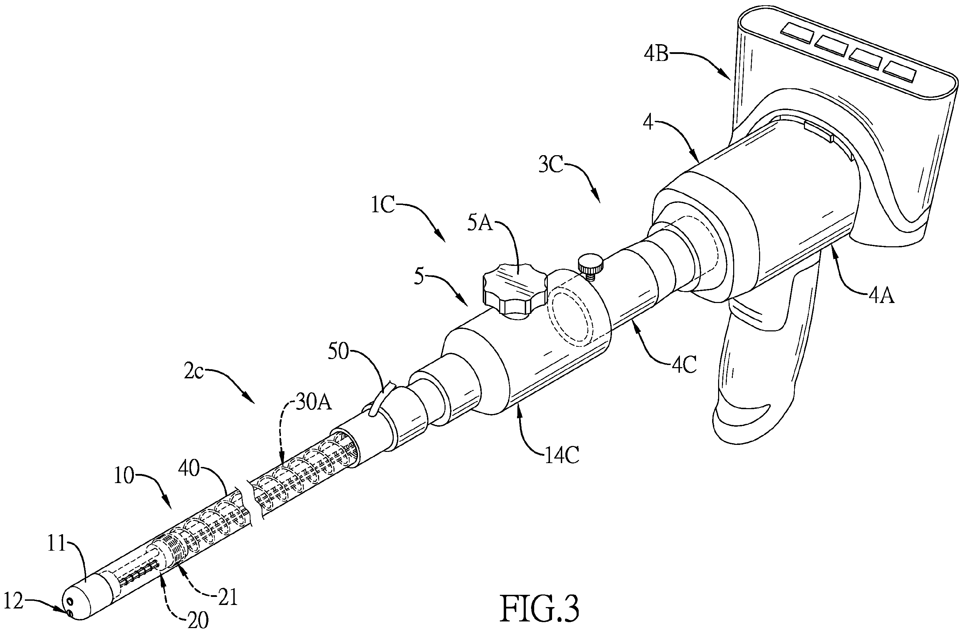

With reference to Fig. 3, a third embodiment of the endoscope 1C has the inspection accessory 2c and the operating set 3C. In the third embodiment, the inspection accessory 2c is substantially same as the inspection accessory 2A of the first embodiment.

In the third embodiment, the inspection accessory 2c has the wire operating unit 5 having a linkage component 14C connected to the fastening component 14A and an adaptor disposed at a rear end of the linkage component 14C and electrically connected to the cable set 13. The linkage component 14C is connected to the connecting portion 4C of the main body 4. The knob 5A is connected to the linkage component 14C. One of the two opposite ends of each one of the multiple angulation wires 22 is connected to the objective optical unit 12. The other one of the two opposite ends of each one of the multiple angulation wires 22 extends inside the linkage component 14C and is connected to the knob 5A. The wire operating unit 5 may be detachably connected to the main body 4 and be disposed with the inspection accessory 2A.

## Claims

1. An endoscope with an inspection accessory (2A, 2B, 2C, 2b, 2c), **characterized in that** the inspection accessory (2A, 2B, 2C, 2b, 2c) comprises:
a distal end cap (11);
a fastening component (14A);
a flexible shaft (30A, 30B, 30C) being telescopic, flexible, and hollow, and having
a first end connected to the fastening component (14A);
a second end opposite to the first end of the flexible shaft (30A, 30B, 30C); and
multiple elemental parts connected in sequence and each one of the multiple elemental parts capable of moving relative to one another;
an objective optical set (10) having
an objective optical unit (12) mounted within the distal end cap (11); and
a cable set (13) electrically connected to the objective optical unit (12);
a bending mechanism (20) connected to the distal end cap (11) and having
a bending component (21);
wherein the second end of the flexible shaft (30A, 30B, 30C) is connected to one of the the distal end cap (11) and the bending component (21); and
a sheath (40) sleeved on the flexible shaft (30A, 30B, 30C) and having two opposite ends;
wherein one of the two opposite ends of the sheath (40) is tightly connected to the distal end cap (11); and
the other one of the two opposite ends of the sheath (40) is tightly connected to the fastening component (14A); and
a conducting tube (50) having a front end and a rear end opposite to the front end of the conducting tube (50);
wherein the front end of the conducting tube (50) is mounted in the distal end cap (11) and communicates with an exterior of the distal end cap (11); and
the rear end of the conducting tube (50) extends out of the fastening component (14A).

2. The endoscope as claimed in claim 1, wherein the flexible shaft (30A) is made up of a spiral thread in a configuration of a compression spring, and has multiple coils connected in sequence and separately disposed.

3. The endoscope as claimed in claim 1, wherein the flexible shaft (30B) is made up of a spiral thread in a configuration of an extension spring, and has multiple coils connected in sequence and tightly disposed and abutting one another.

4. The endoscope as claimed in claim 1, wherein the flexible shaft (30C) has multiple elemental strips sequentially connected to one another and capable of moving relative to one another.

5. The endoscope as claimed in any one of claims 1 to 4, wherein the objective optical set (10) having a connector element disposed within the fastening component (14A) and electrically connected to the cable set (13).

6. The endoscope as claimed in any one of claims 1 to 4, wherein
the inspection accessory (2A, 2B, 2C, 2b, 2c) has
a wire operating unit (5) connected to the fastening component (14A) and having a linkage component (14C) and a knob (5A) connected to the linkage component (14C) and capable of rotating;
the bending mechanism (20) has multiple angulation wires (22) extending along the flexible shaft (30A, 30B, 30C);
each of the multiple angulation wires (22) has two opposite ends;
one of the two opposite ends of each one of the multiple angulation wires (22) is connected to the objective optical unit (12);
the other one of the two opposite ends of each one of the multiple angulation wires (22) extends inside the linkage component (14C) and is connected to the knob (5A) of the wire operating unit (5); and
the objective optical set (10) has a connector element disposed within the fastening component (14A) and electrically connected to the cable set (13).

7. The endoscope as claimed in any one of claims 1 to 4, wherein
the objective optical set (10) has a connector element disposed within the fastening component (14A) and electrically connected to the cable set (13);
the endoscope has an operating set (3A) detachably connected to the inspection accessory (2A) and having
a main body (4) having
a base (4A) having a front end and a rear end opposite to each other;
a processing unit disposed within the base (4A);
a display (4B) disposed at the rear end of the base (4A) and electrically connected to the processing unit; and
a connecting portion (4C) disposed at the front end of the base (4A) and connected to the fastening component (14A); and
a connector disposed within the connecting portion (4C) and electrically connected to the processing unit and the connector element inside the fastening component (14A); and
a wire operating unit (5) mounted to the connecting portion (4C) of the main body (4) and having a knob (5A); and
the bending mechanism (20) has multiple angulation wires (22) extending along the flexible shaft (30A, 30B, 30C) and connected to the knob (5A).

8. The endoscope as claimed in any one of claims 1 to 4, wherein
the objective optical set (10) has a connector element disposed within the fastening component (14A);
the endoscope has an operating set (3A) detachably connected to the inspection accessory (2A) and having
a wire operating unit (5) having
a linkage component (14C) connected to the fastening component (14A);
a knob (5A) connected to the linkage component (14C); and
an adaptor disposed at a rear end of the linkage component (14C) and electrically connected to the cable set (13);
the bending mechanism (20) has multiple angulation wires (22) extending along the flexible shaft (30A, 30B, 30C);
each of the multiple angulation wires (22) has two opposite ends;
one of the two opposite ends of each one of the multiple angulation wires (22) is connected to the objective optical unit (12);
the other one of the two opposite ends of each one of the multiple angulation wires (22) extends inside the linkage component (14C) and is connected to the knob (5A);
the operating set (3A) further has
a main body (4) having
a base (4A) having a front end and a rear end opposite to each other;
a processing unit disposed within the base (4A);
a display (4B) disposed at the rear end of the base (4A) and electrically connected to the processing unit; and
a connecting portion (4C) disposed at the front end of the base (4A) and connected to the linkage component (14C); and
a connector disposed within the connecting portion (4C) and electrically connected to the processing unit and the connector element.

9. The endoscope as claimed in any one of claims 1 to 4, wherein the flexible shaft (30A, 30B, 30C) is made of steel.
